# EUROPEAN PATENT SPECIFICATION

(11) **EP 4 196 104 B1**
(45) Date of publication and mention of the grant of the patent: **11.12.2024**
(21) Application number: 21766135.4
(22) Date of filing: 13.08.2021
(51) Int. Cl.: A61K 31/132, A61K 31/352, A61K 31/7048, A61K 36/00, A61P 31/12, A61K 36/61, A61P 31/14, A61P 31/16, A61P 31/22

(54) **ORAL PRODUCT COMPRISING AN ANTIVIRAL ACTIVE INGREDIENT**
ORALES PRODUKT MIT EINEM ANTIVIRALEN WIRKSTOFF
PRODUIT ORAL COMPRENANT UN PRINCIPE ACTIF ANTIVIRAL

(30) Priority: 14.08.2020 WO PCT/EP2020/072935
(43) Date of publication of application: 21.06.2023
(73) Proprietor: PFAB, Florian, 82166 Gräfelfing (DE)
(72) Inventor: PFAB, Florian, 82166 Gräfelfing (DE)
(74) Representative: Hoffmann Eitle
(86) International application number: PCT/EP2021/072653
(87) International publication number: WO 2022/034234

(56) References cited:
- WO-A1-2016/003313
- WO-A1-2021/205029
- WO-A2-2010/081204
- CN-A- 103 230 421
- CN-A- 111 407 859
- IT-A1- MI20 102 308
- KR-B1- 101 627 065
- NILS C. GASSEN ET AL: "Analysis of SARS-CoV-2-controlled autophagy reveals spermidine, MK-2206 and niclosamide as putative antiviral therapeutics", BIORXIV, 15 April 2020 (2020-04-15), XP055760013, Retrieved from the Internet <URL:https://www.biorxiv.org/content/10.1101/2020.04.15.997254v1> DOI: 10.1101/2020.04.15.997254
- RUBEN MANUEL LUCIANO COLUNGA BIANCATELLI ET AL: "Quercetin and Vitamin C: An Experimental, Synergistic Therapy for the Prevention and Treatment of SARS-CoV-2 Related Disease (COVID-19)", FRONTIERS IN IMMUNOLOGY, vol. 11, 1 January 2020 (2020-01-01), pages 1451, XP055759945, DOI: 10.3389/fimmu.2020.01451
- KEN WATANABE ET AL: "Anti-influenza Viral Effects of Honey In Vitro: Potent High Activity of Manuka Honey", ARCHIVES OF MEDICAL RESEARCH, vol. 45, no. 5, 1 July 2014 (2014-07-01), MX, pages 359 - 365, XP055759939, ISSN: 0188-4409, DOI: 10.1016/j.arcmed.2014.05.006

## Description

### Field of the Invention

The present invention relates to an oral product comprising an antiviral active ingredient for use in a method of treatment or prevention of an infection with a coronavirus, an influenza virus, a coxsackievirus, or a herpes virus. The active ingredient of this oral product includes a combination of spermidine and one or more polyphenols.

### Description of the Related Art

**Coronaviruses** are single stranded (+) ribonucleic acid (RNA) viruses of the *Coronaviridae* family. Coronaviruses can infect both humans and animals and can cause respiratory infections such as severe acute respiratory syndrome (SARS), Middle East respiratory syndrome (MERS), avian infectious bronchitis virus (IBV) and COVID-19. Coronaviruses capable of infecting humans include the alphacoronaviruses (αCoVs) HCoV-NL63 (Human CoV-NL63) and HCoV-229E, and the betacoronaviruses (βCoVs) HCoV-OC43 (Human CoV-OC43), HKU1 (Human CoV), SARS-CoV (Severe Acute Respiratory Syndrome CoV), MERS-CoV (Middle Eastern Respiratory Syndrome CoV), and SARS-CoV-2 (Severe Acute Respiratory Syndrome CoV-2).

Coronavirus infection is very common. There are presently no antiviral drugs or vaccines to prevent or treat many of the coronavirus strains, in particular SARS-CoV-2 responsible for coronavirus disease 2019 (COVID-19). The spread of COVID-19 has led to the 2019-20 coronavirus pandemic.

**Influenza viruses** are enveloped, negative-strand RNA viruses with a segmented genome and are members of the *Orthomyxoviridae* family. Influenza virus A and B cause influenza in humans.

**Coxsackievirus** is a single-stranded RNA virus and belongs to the *Picornaviridae* family. Coxsackievirus causes coxsackievirus infection such as hand-foot-mouth disease. Coxsackieviruses are subdivided into two serogroups, A and B.

**Herpes viruses** have a double-stranded DNA genome and belong to the family *Herpesviridae.* Herpes viruses, such as herpes simplex virus (HSV), varicella-zoster virus (VZV) or human cytomegalovirus (HCMV), cause several diseases in humans and animals. Herpes simplex virus includes herpes simplex virus type 1 (HSV-1) and 2 (HSV-2). Varicella-zoster virus (VZV) causes chickenpox (varicella) and shingles (herpes zoster).

Coronavirus, influenza virus, coxsackievirus, and herpes virus are known to be transmitted via various routes including via the oral cavity. Human-to-human transmission of coronavirus can occur through direct transmission, such as droplet inhalation, i.e. aerosol inhalation, and contact transmission, such as the contact with oral, nasal, and eye mucous membranes. Influenza can infect a subject by entering the nasal or respiratory tract. For example, a virus may be released from an infected host by sneezing, coughing or talking. There are many routes of transmission of coxsackievirus through contact with infective secretions or excretions. Herpes simplex virus is mainly transmitted by oral-to-oral contact. Human cytomegalovirus (HCMV) is transmitted via body fluids such as saliva. VZV can be transmitted through aerosols or direct contact with the virus in lesions and infects the respiratory mucosal epithelium.

**Spermidine** is active against diseases caused by herpes simplex virus. WO93/12777 discloses that spermidine has a neuroprotective effect, in particular as a cationic channel regulating agent. Nils C. Gassen ET AL: "Analysis of SARS-CoV-2-controlled autophagy reveals spermidine, MK-2206 and niclosamide as putative antiviral therapeutics",bioRxiv, 15 April 2020 (2020-04-15) discloses an vitro study on the use of spermidine to treat SARS-CoV-2 infections.

**Quercetin** is a plant flavonoid and is known to have antiviral properties. Mani, J. S., Johnson, J. B., Steel, J. C., Broszczak, D. A., Neilsen, P. M., Walsh, K., Naiker, M., Virus Research, 2020, 284, 197989, discloses that quercetin is a potential inhibitor of coronavirus in humans. Bachevski, D., Damevska, K., Simeonovski, V., Dimova, M., Dermatologic Therapy, 2020, discloses that quercetin and its derivatives inhibit the SARS-CoV-1 and MERS-CoV main protease *in vitro.* It is postulated that quercetin may have anti-coronavirus effects through its modulation of the cellular unfolded protein response (UPR) which is utilised by coronaviruses to complete their entire replication cycle.

RUBEN MANUEL LUCIANO COLUNGA BIANCATELLI ET AL: "Quercetin and Vitamin C: An Experimental, Synergistic Therapy for the Prevention and Treatment of SARS-CoV-2 Related Disease (COVID-19) discloses that quercetin is active against SARS-CoV-2.

Kim, C. H., Kim, J.-E., Song, Y.-J., Molecules, 2020, 25, 2379, discloses that quercetin and isoquercetin have antiviral activity against human VZV and HCMV and effectively inhibit human herpes virus replication. It is also known that quercetin and isoquercetin have antiviral activities against herpes simplex virus type 1 (HSV-1) and 2 (HSV-2). Quercetin and isoquercetin suppress NF-κB activation in HSV-1-infected cells. Quercetin is also known to reduce HSV-1-induced interferon (IFN) regulatory factor 3 (IRF3) activation or to inhibit HSV-1 entry into host cells. Biancatelli, R. M. L. C., Berrill, M., Catravas, J. D., Marik, P. E., Front Immunol., 2020, 11, 1451 discloses that quercetin inhibits the cytopathic effects provoked by coxsackievirus (A21 and B1). **Propolis** is known from Bachevski, D., Damevska, K., Simeonovski, V., Dimova, M., Dermatologic Therapy, 2020 to have antiviral properties and to be a possible inhibitor of SARS-CoV-2 in the oropharyngeal niche. Alcoholic or aqueous propolis extracts have been shown to possess a broad-spectrum antiviral activity against viruses including DNA viruses such as HSV-1, HSV-2, and RNA viruses such as Influenza virus type A and B.

**Manuka-honey** is disclosed in Watanabe, K., Rahmasari, R., Matsunaga, A., Haruyama, T., Kobayashi, N., Archives of Medical Research, 2014, 45, 359-365, to have antiviral activities, in particular against influenza viruses and VZV. It was found that manuka honey efficiently inhibited influenza virus replication.

Some **essential oils** are known to have antiviral activities, in particular the essential oils tea tree, sage, cinnamon, eucalyptus, oregano, thyme, basil, cassia, lime, lemon, peppermint, melissa, clove, and lavender. Melissa, a lamiaceae plant, is known to be efficacious against the herpes virus. Australian Tea Tree oil, clove essential oil, peppermint essential oil, thyme essential oil, basil essential oil, lavender essential oil, oregano essential oil, and eucalyptus essential oil (see Ghasemiana, A., Eslamib, M., Hasanvandc, F., Bozorgid, H., Al-abodie, H.R., Comparative Immunology, Microbiology and Infectious Diseases 65, 2019, 234-237) are also known to have an antiviral effect against herpes simplex virus. Sage essential oil is known to be active against SARS-CoV. Cinnamon essential oil is known to be active against influenza virus. Basil essential oil is known to show antiviral activity against coxsackievirus.

It is known to use oral products, such as chewing gums, as a vehicle for delivering components, such as medicaments, to the oral cavity. For example, chewing gums with the active ingredients caffeine and nicotine are known. It is also known that medicated oral products can release an active ingredient in the oral cavity for making it systemically available or for acting locally in the oral cavity.

An object of the present invention is to provide an improved oral product for use in a method of treatment or prevention of an infection with a coronavirus, an influenza virus, a coxsackievirus, or a herpes virus.

### Summary of the Invention

The present invention relates to an oral product designed to release an antiviral active ingredient within the oral cavity of a user. The release of the antiviral active ingredient within the oral cavity of a user may treat or prevent viral infection.

In particular, the present invention relates to an oral product comprising an antiviral active ingredient which includes a combination of spermidine and one or more polyphenols, for use in a method of treatment or prevention of an infection with a coronavirus, an influenza virus, a coxsackievirus, or a herpes virus, wherein the method comprises the administration of the oral product to the oral cavity, the active ingredient is released from the oral product into the oral cavity, and the treatment or prevention involves reducing the virus load in the respiratory tract, and in the exhaled air.

Preferred embodiments of the oral product of the present invention are defined in the dependent claims.

### Detailed Description and Preferred Embodiments

Hereinafter, the invention will be described in detail.

### Oral products

The oral product of the present invention comprises an antiviral active ingredient which includes a combination of spermidine and one or more polyphenols, such as quercetin and isoquercetin.

The oral product of the present invention contains an effective amount of the antiviral active ingredient. An effective amount is an amount sufficient to kill or inactivate the respective viruses. One of ordinary skill in the art will be able to adjust the dosing regimen of the antiviral active ingredients used in present invention to achieve the desired therapeutic effect in a particular user. The user may use the oral product repeatedly throughout the day.

In a further preferred embodiment, the oral product of the present invention comprises a combination of spermidine, polyphenols, such as quercetin and isoquercetin, propolis, and manuka-honey as antiviral active ingredient.

In another preferred embodiment, the oral product of the present invention comprises a combination of spermidine, polyphenols, such as quercetin and isoquercetin, propolis, manuka-honey, and essential oils as antiviral active ingredient.

In another preferred embodiment, the oral product of the present invention comprises a combination of spermidine, polyphenols, such as quercetin and isoquercetin, propolis, manuka-honey, natural extracts, and essential oils as antiviral active ingredient.

The amount of antiviral active ingredients in the oral product may be 0.1 to 50, preferably 1 to 25, 1 to 15, 1 to 12, 5 to 15 or 5 to 10, and more preferably 5 to 10 or 5, 10, 12 or 15 weight % of the total composition.

Any amount indicated in percent herein relates to weight percent relative to the total weight of the respective oral product, such as e.g. a chewing gum, which amounts to 100%, unless stated otherwise.

In one embodiment, the oral product is formulated to deliver at least about 1 mg to about 400 mg of the active ingredient to the oral cavity, preferably about 10 mg to about 200 mg, and more preferably about 20 mg to about 100 mg. In one embodiment, a typical oral product, such as a chewing gum or a lozenge, weighing about 0.5 to about 4.5 g may contain about 1 mg to about 400 mg of the active ingredient.

The formulation of the oral product can vary depending on the desired masticatory and other sensory characteristics of the final product.

The oral product may be a product selected from mouth soluble or dispersible forms; suckable, eatable, chewable or coherent forms; or forms rapidly disintegrating in the mouth.

The oral product can have a variety of sizes and shapes including disk, rectangle, and square. The oral products can be packaged in a variety of conventional ways. The oral product can be consumed by people of all ages.

The oral product may comprise a body that is at least partially receivable in the oral cavity. The oral product may comprise a body that is wholly receivable in the oral cavity. The oral product can be attached to a rod, tube, or stick which is reuseable or a single use product. The tip piece can include the oral product. The rod, tube, or stick can act as a holder for the tip piece. The tip piece can be shaped to be at least partially received within the oral cavity.

The oral product may dissolve or disintegrate when exposed to saliva within the oral cavity. Alternatively, the oral product is kept in the oral cavity for chewing or sucking or slowly dissolving in the oral cavity and may be discarded after use.

The oral product is selected from a chewing gum, a lozenge, a pastille, a gel, granules, a capsule, a tablet, a toffee, and hard boilies. The oral product is preferably a chewing gum, a lozenge, a pastille, a gel, a tablet, or hard boilies, and more preferably a chewing gum or lozenge. The oral product is most preferably a chewing gum.

The **chewing gum** may include a gum base. The gum base portion is retained in the mouth throughout the chew. After use, the chewing gum can be removed from the oral cavity and discarded. The gum base may include any component known in the chewing gum art. The person skilled in the art understands that the components of the gum base provide the viscoelastic properties. Such components may be water soluble, water-insoluble or a combination thereof. A gum base may include elastomers, bulking agents, waxes, elastomer solvents, emulsifiers, plasticisers, fillers, and combinations thereof. The gum base may constitute 5% to 95% by weight of the chewing gum, preferably 10% to 80%, more preferably 20% to 70%, 70% to 90% or 80% to 90% and most preferably 40%, 50%, 60%, 70%, 80% or 90%.

The chewing gum may further include plasticisers or softeners to improve consistency during chew, increase flexibility, and optimise mouthfeel of the gum. The chewing gum may also include fillers and bulking agents. Softeners are typically used in amounts of about 5 or 15% to about 40 or 80% by weight of the chewing gum base.

The chewing gum may be prepared in the form of a slab, pellet, stick, multi-layer, centre-filled gum, liquid-filled gum, extruded, deposited, compressed chewing gum or any other suitable format, optionally with the addition of magnesium stearate as compressing aid. When used as compressing aid magnesium stearate is added in an amount of 0.5% to 10%, preferably 1% to 5% and most preferably 2%to3% based on the total composition of the chewing gum.

The chewing gum formulation should be able to maintain masticability for extended periods of time.

In one embodiment, the chewing gum comprises a gum base, a sweetening agent, and a flavourant.

In one embodiment, the chewing gum may contain one or more sweetening agents. The amount of the one or more sweetening agents in the chewing gum may be 0.01% to 10%, preferably 0.1% to 5%, more preferably 0.2% to 2%, of the chewing gum.

In another embodiment, the oral product may contain one or more flavourants. The amount of the one or more flavourants in the chewing gum may be 0.1% to 10%, preferably 0.5% to 5% of the chewing gum.

The chewing gum may be coated or not coated. The active ingredient may be included in the coating. A suitable coating may be a hard coating, soft coating, a film coating or a sealing coating or a combination thereof. Coating substances may include, but are not limited to, sorbitol, maltitol, mannitol, xylitol, erythritol, lactitol, maltose, fructose, xylose, erythrose, lactose, isomaltulose, D-galactose, or any combinations thereof.

A **lozenge** may include sweetening agents or flavourants, or combinations thereof.

A **pastille** may include a plasticiser, gelling agent, sweetening agent, a flavourant, and a lubricant to prevent sticking between the die wall and the pastille.

A **gel** may be a sticky gel. A gel may include a gel carrier such as poloxamer gel or a methyl cellulose gel.

The oral product may be a **tablet,** wherein the tablet may be chewable or orally disintegrating. A tablet may include binders, sweetening agents, and flavourants.

**Hard boilies** are a mixture of sugar and other carbohydrates. They can be in an amorphous or glassy condition.

### Antiviral agents

The following compounds can be used as antiviral agents in the oral products of the present invention: spermidine, polyphenols, such as quercetin and isoquercetin, propolis, manuka-honey, natural extracts, and essential oils. The amount of the antiviral active ingredients in total in the oral product may be 0.1 to 50, preferably 1 to 25, 1 to 15, 1 to 12, 5 to 15 or 5 to 10, and more preferably 5 to 10 or 5, 10, 12 or 15 weight % of the total composition.

Spermidine is a polyamine. Spermidine for use in the invention preferably is derived from wheat germ. It can be used in the form of its pharmacologically acceptable salts. The amount of spermidine may be 0.001% to 2%, preferably 0.01% to 1%, more preferably 0.02% to 0.5%, 0.02% to 0.1%, or 0.05% of the total composition of the oral product.

Quercetin and isoquercetin (also known as quercetin 3-O-β-D-glucoside) are polyphenols. The amount of quercetin and/or isoquercetin may be 0.1% to 10%, preferably 0.5% to 5%, more preferably 1% to 3%, or 1.5% of the total composition of the oral product.

The total amount of spermidine and quercetin may be 0.1% to 10%, preferably 0.5% to 5%, more preferably 1% to 3% and most preferably 1.55% of the total composition of the oral prod uct.

Propolis is a complex natural resinous mixture produced by honey-bees (*Apis mellifera*), and includes honey, resins, waxes, and pollen. The composition is dependent on the geographical origin, climate, water availability, and other environmental factors. Propolis may be present in an amount of 0.1% to 10%, preferably 0.5% to 5%, more preferably 1% to 3% of the total composition of the oral product.

Manuka-honey is a natural product produced by bees. The composition is variable due to the source on which the bees feed, and the major components are fructose, glucose, and sucrose. Manuka-honey may be present in the oral product in an amount of 0.1% to 10%, preferably 0.5% to 5%, more preferably 1% to 3% of the total composition of the oral product.

Essential oils are aromatic, volatile liquids, which can be natural (i.e., derived from plants), or synthetic. They can be extracted from organic material such as plants. They can be derived from the seeds, berries, flowers, fruits, leaves, stalks, barks, wood, roots, and rhizomes of plant sources including, but not limited to, one or more of tea tree, sage, cinnamon, ginger, eucalyptus, oregano, thyme, basil, cassia, lime, lemon, peppermint, melissa, clove or lavender, and combinations thereof. Essential oils can be extracted from plants by distillation, e.g. steam distillation.

The term "essential oils" refers to polychemical blends which include a number of different chemical species, such as 2 to 50 chemical species, including, but not limited to, monoterpenes, sesquiterpenes, diterpenes, triterpenes, terpene alcohols, terpenoids, phenylpropanoids, ethers, phenols, aldehydes, esters, ketones, alcohols, and oxides. The composition of natural essential oils will depend on the particular species from which they were extracted and will also depend on geographical origin, climate and other environmental factors.

Tea tree essential oil may be derived from *Melaleuca alternifolia* and may comprise the components terpinen-4-ol, γ-terpinene, α-terpinene, or any combination thereof. Sage essential oil may be derived from plants of the *Salvia genus* and may comprise the components 1,8-cineole, camphor, α-thujone, β-thujone, borneol, viridiflorol, or any combination thereof. Cinnamon essential oil may be derived from plants of the *Cinnamomum genus* and may comprise the component cinnamaldehyde. Ginger essential oil may be derived from *Zingiber officinale* and may comprise saponins also referred to as triterpene glycosides. Eucalyptus essential oil may be derived from plants of the *Eucalyptus genus* and may comprise the components 1,8-cineol or α-pinene, or both. Oregano essential oil may be derived from plants of the *Origanum genus* and may comprise carvacrol or thymol, or both. Thyme essential oil may be derived from plants of the *Thymus genus* and may comprise the components thymol, p-cymene, γ-terpinene, or any combination thereof. Basil essential oil may be derived from *Ocimum basilicum* and may comprise the component methyl eugenol. Cassia may be derived from *Cinnamomum cassia* and may comprise the component cinnamaldehyde. Lime essential oil may be derived from *Citrus* and may comprise the components limonene, γ-terpinene, β-pinene, or any combination thereof. Lemon essential oil may be derived from *Citrus* and can comprise the components limonene or β-pinene, or both. Peppermint essential oil may be derived from plants of the *Mentha genus* and may comprise the components menthol, menthone, menthofuran, 1,8-cineole, menthyl acetate, or any combination thereof. Melissa essential oil may be derived from *Melissa officinalis* L. (Family of Lamiaceae) and may comprise the components geranial or neral, or both. Clove essential oil may be derived from *Eugenia* caryophyllus and may comprise the component eugenol. Lavender essential oil may be derived from plants of the *Lavandula genus* and may comprise the components linalool, linalyl acetate, lavandulol, geraniol, eucalyptol, or any combination thereof.

In a preferred embodiment, the essential oils are selected from essential oils from tea tree, sage, cinnamon, ginger, eucalyptus, peppermint, and clove.

The oral product may contain one essential oil or more essential oils in combination. Preferred essential oils include essential oils from tea tree, sage, cinnamon, ginger, eucalyptus, peppermint, clove, or any combinations thereof. Another particularly preferred combination of essential oils includes essential oils from cinnamon, ginger and peppermint. Further preferred is a combination of essential oil which includes essential oils from lemon, cinnamon, ginger and peppermint.

In one embodiment, the essential oil or the combination of essential oils may be present in a total amount of 0.5% to 20%, preferably 1% to 10%, more preferably 3% to 7% and most preferably 5% or 6% of the total composition of the oral product.

In preferred embodiment, the essential oil is provided as a combination of essential oils which comprise essential oils from lemon, cinnamon, ginger and peppermint, wherein the essential oils from cinnamon, ginger and peppermint may be present in a total amount of 0.5% to 20%, preferably 1% to 15%, more preferably 3% to 10% and most preferably 5.5% of the total composition of the oral product.

The term natural extract means any extract that is obtained from a natural source, such as a plant. The natural extract may be from tea tree, sage, cinnamon, ginger, ginseng, eucalyptus, oregano, thyme, basil, cassia, lime, lemon, peppermint, melissa, clove or lavender, preferably from tea tree, sage, cinnamon, ginger, ginseng, eucalyptus, peppermint, and clove, and more preferably from lemon, cinnamon, ginger and peppermint or cinnamon, ginger and peppermint. The natural extract can be derived from the same sources as discussed above for the corresponding essential oils and may comprise the same components.

A natural extract derived from ginseng (*Panax Ginseng*) may be derived from dried roots and may comprise saponins.

The oral product may contain one natural extract or more natural extracts in combination. In one embodiment, the natural extract or the combination of natural extracts may be present in a total amount of 0.1% to 20%, preferably 1% to 10%, more preferably 2% to 5% and most preferably 2% or 4% of the total composition of the oral product.

In a preferred embodiment, the natural extract is derived from ginseng. It may be present in an amount of 0.1% to 10%, preferably 0.5% to 5% and more preferably 2% to 4% of the total composition of the oral product.

In another preferred embodiment, the oral product may contain one or more natural extract(s) in combination with one or more essential oil(s). In one embodiment, the total amount of the one or more natural extract(s) and the one or more essential oil(s) may be 1% to 40%, preferably 20-30% by weight of the total composition of the oral product.

### Excipients

**Elastomers** may be any water-insoluble polymer known in the art. The elastomers include natural and synthetic elastomers. Natural elastomers may include, but are not limited to, chicle gum, natural rubber such as smoked or liquid latex, crown gum, sorva, rosidinha, jelutong, perillo, nispero, niger gutta, tunu, balata, guttapercha, lechi caspi, gutta kay, or any combinations thereof. Synthetic elastomers may include, but are not limited to, polyethylene, styrene-butadiene copolymers (SBR), isobutylene-isoprene copolymers, polyisobutylene, polyvinyl acetate, or any combinations thereof. The oral product may contain one or more elastomers. In one embodiment, the one or more elastomers may be present in a total amount of 1% to 30% and preferably 5% to 10% of the total composition of the oral product.

**Elastomer solvents** (also known as elastomer plasticisers) may include, but are not limited to, pentaerythritol ester of partially hydrogenated wood rosin, pentaerythritol ester of wood rosin, glycerol ester of polymerised rosin, glycerol ester of partially dimerized rosin, glycerol ester of tall oil rosin, glycerol ester of wood rosin and partially hydrogenated wood rosin and partially hydrogenated methyl ester of rosin, terpene resins, polymers of α-pinene or β-pinene, or any combinations thereof. The oral product may contain one or more elastomer solvents. In one embodiment, the one or more elastomer solvents may be present in a total amount of 1% to 30% and preferably 5% to 10% of the total composition of the oral product.

**Plasticisers** may include, but are not limited to, glycerin, propylene glycol, vegetable oil, and medium chain triglycerides, or any combinations thereof. The oral product may contain one or more plasticisers. In one embodiment, the one or more plasticizers may be present in a total amount of 0.1% to 10% and preferably 2% to 5% of the total composition of the oral product.

**Softeners** may include, but are not limited to, lecithin, tallow, hydrogenated tallow, hydrogenated and partially hydrogenated vegetable oils, glycerol monostearate, cocoa butter, glycerol triacetate, mono- and triglycerides, acetylated monoglycerides, fatty acids, or any combinations thereof. The oral product may contain on or more softeners. In one embodiment, the one or more softeners may be present in amounts of about 5 or 15% to about 40 or 80% by weight of the total composition of the oral product.

**Fillers** may be mouth stable or can dissolve or disintegrate during use. Fillers may include magnesium and calcium carbonate, talc, ground limestone, sodium sulfate, lactose, mannitol, sorbitol, microcrystalline cellulose, mono-, di- and tri-calcium phosphate, magnesium and aluminium silicate, kaolin and clay, aluminium oxide, titanium oxide, cellulose polymers, or any combinations thereof. The oral product may contain one or more fillers. In one embodiment, the one or more fillers may be present in a total amount of 0.1% to 10% and preferably 2% to 5% of the total composition of the oral product.

**Waxes** may include, but are not limited to, petroleum wax, microcrystalline wax, natural wax, paraffin wax, or any combinations thereof. The oral product may contain one or more waxes. In one embodiment, the one or more waxes may be present in a total amount of 0.1% to 10% and preferably 2% to 5% of the total composition of the oral product.

**Lubricants** may include, but are not limited to, lecithin. The oral product may contain one or more lubricants. In one embodiment, the one or more lubricants may be present in a total amount of 0.1% to 10% and preferably 2% to 5% of the total composition of the oral product.

**Buffering agents** may include, but are not limited to, amino acid buffers, dipotassium phosphate, glycerophosphate, carbonate, including monocarbonate, bicarbonate or sesquicarbonate, glycerinate, acetate, glyconate or citrate of an alkali metal, such as potassium and sodium, ammonium, or any combinations thereof. The oral product may contain one or more buffering agents. In one embodiment, the one or more buffering agents may be present in a total amount of 0.1% to 10% and preferably 2% to 5% of the total composition of the oral prod uct.

**Stabilising agents** may include, but are not limited to, ascorbic acid, monosterol citrate, butylated hydroxytoluene (BHT), butylated hydroxyanisole (BHA), or any combinations thereof. The oral product may contain one or more stabilising agents. In one embodiment, the one or more stabilising agents may be present in a total amount of 0.1% to 10% and preferably 2% to 5% of the total composition of the oral product.

**Gelling agents** may include, but are not limited to, gelatin, carrageenan, or any combinations thereof. The oral product may contain one or more gelling agents. In one embodiment, the one or more gelling agents may be present in a total amount of 0.1% to 10% and preferably 2% to 5% of the total composition of the oral product.

**Preservatives** may include, but are not limited to, methyl paraben, propyl paraben, sodium methyl paraben, sodium propyl paraben, or any combinations thereof. The oral product may contain one or more preservatives. The one or more preservatives may be present in a total amount of 0.1% to 10% and preferably 2% to 5% of the total composition of the oral product.

**Adsorbants** may include, but are not limited to, talc, clay, silicone dioxide, activated charcoal, calcium silicate, maltodextrin, magnesium aluminium silicate, natural starches, cyclodextrin, or any combinations thereof. The oral product may contain one or more adsorbants. In one embodiment, the one or more adsorbants may be present in a total amount of 1% to 20%, preferably 5% to 15%, more preferably 6% to 12% of the total composition of the oral product.

**Additives** may include sweetening agents, flavourants, colourants, taste masking agents, oral care agents, minerals, vitamins, amino acids, antioxidants, and therapeutic agents. The oral product may contain one or more additives. In one embodiment, the one or more additives may be present in a total amount of 0.1% to 20%, preferably 0.5% to 10 % and more preferably 2% to 5% of the total composition of the oral product.

**Sweetening agents** may include, but are not limited to, bulk sweeteners, sugar sweeteners, sugar substitute sweeteners, artificial sweeteners, high-intensity sweeteners, or any combinations thereof. The sweetening agents may include, but are not limited to, monosaccharides, di-saccharides, and poly-saccharides such as glucose, sucrose, dextrose, dextrin, maltose, xylose, ribose, lactose, mannose, galactose, fructose, levulose, fructo oligo saccharide syrups, partially hydrolysed starch, corn syrup and their solids, isomaltulose, or any combinations thereof. Sugarless sweeteners include, but are not limited to, sugar alcohols such as maltitol, sorbitol, mannitol, xylitol, hydrogenated starch hydrolysates, or any combinations thereof. Bulk sweeteners include both sugar and sugarless components. High intensity sweeteners include, but are not limited to aspartame, alitame, sucralose, salts of acesulfame, saccharin and its salts, cyclamic acid and its salts, glycyrrhizin, dihydrochalcones, thaumatin, monellin, sterioside, or any combinations thereof. The amount of sweetening agent in the oral product may be about 0.1% to 40%, preferably 1% to about 10% or to 5% of the total composition.

**Flavourants** may include natural and synthetic flavours. Flavourants may include essential oils, natural extracts, synthetic flavours, essences, powders, acids, or any combinations thereof. More specifically, flavourants may include, but are not limited to oils selected from spearmint oil, oil of wintergreen, cinnamon oil, peppermint oil, clove oil, bay oil, anise oil, eucalyptus oil, thyme oil, sage oil, cedar leaf oil, Japanese mint oil, oil of nutmeg, allspice, mace, oil of bitter almonds, and cassia oil. Alternatively, flavourants may include, but are not limited to aromas selected from bourbon, scotch, whiskey, coconut, coffee, chocolate, vanilla, grape fruit, orange, lime, menthol, liquorice, lavender, cinnamon, chamomile, cardamom, clove, caramel aroma, honey aroma, peanut, walnut, cashew, hazelnut, almonds, pineapple, strawberry, banana, mango, pomegranate, papaya, raspberry, apple, pear, peach, ginger, apricot, blackberry, cherry, plum essence, anise, thyme, nutmeg, peppermint, wintergreen, spearmint, eucalyptus, mint, watermelon, or any combinations thereof. The flavourants may be used in liquid or solid form. The oral product may contain one or more flavourants. In one embodiment, the one or more flavourants may be present in a total amount from about 0.02% to about 20%, preferably 0.1% to 10% or 2% to 5% of the total composition.

**Colourants** may include natural and artificial food colours and dyes suitable for food. In particular, colourants may include fruit and vegetable extracts. Artificial colourings include Fast green (E143), Brilliant blue (E133), Allura red (E129), Erythrosine (pink) (E127), tartrazine (E102), indigotine (E132), and Sunset yellow (El 10). The colourant may be present in amounts of up to about 6% by weight of the composition. The oral product may contain one or more colourants. In one embodiment, the one or more colourants may be present in a total amount of 0.01% to 10% and preferably 2% to 5% of the total composition.

**Taste masking agents** may include, but are not limited to, menthol or peppermint oil. The oral product may contain one or more taste masking agents. In one embodiment, the one or more taste masking agents may be present in a total amount of 0.01% to 10% and preferably 2% to 5% of the total composition.

**Oral care agents** that may be used in the oral product include those actives known to the skilled person, such as anti-plaque agents, breath freshening agents, anti-microbial agents, antibacterial agents, anti-calculus agents, tooth whitening agents, oral malodor control agents, fluoride compounds, quaternary ammonium compounds, remineralization agents, or any combinations thereof. The oral product may contain one or more oral care agents. In one embodiment, the one or more oral care agents may be present in a total amount of 0.01% to 10% and preferably 2% to 5% of the total composition.

**Minerals** may include, but are not limited to, calcium (e.g. as carbonate, citrate), chromium (e.g. as picolinate), magnesium (e.g. as oxide), iron (e.g. as bisglycinate), zinc (e.g. as bisglycinate) or any combinations thereof, preferebly zinc bisglycinate. The oral product may contain one or more minerals. In one embodiment, the one or more minerals may be present in a total amount of 0.01% to 10%, preferably 0.1% to 5%, more preferably 0.2% to 2% of the total composition.

**Vitamins** may include, but are not limited to, vitamins A, B, such as B1, B2 or B6, C, D, such as D2 or D3, E, F, K, or any combinations thereof. A preferred vitamin is vitamin D. The oral product may contain one or more vitamins. In one embodiment, the one or more vitamins may be present in a total amount of 0.0001% to 1% and preferably 0.0002% to 0.001% of the total composition.

**Amino acids** may include, but are not limited to, proteinogenic amino acids, i.e. alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine, and non-proteinogenic amino acids, such as, for example, taurine, gamma-aminobutyric acid (GABA), lanthionine, 2-aminoisobutyric acid, dehydroalanine, carnitine, ornithine and citrulline. The oral product may contain one or more amino acids. In one embodiment, the one or more amino acids may be present in a total amount of 0.1% to 10% and preferably 2% to 5% of the total composition.

**Antioxidants** may include, but are not limited to, ascorbyl palmitate, butylated hydroxytoluene, sodium ascorbate, ascorbic acid, monosterol citrate, tocopherols, tertiary butylhydroquinone (TBHQ), butylated hydroxyanisole (BHA), propyl gallate, Vitamin E, and derivatives thereof. The oral product may contain one or more antioxidants. In one embodiment, the one or more antioxidants may be present in a total amount of 0.1% to 10% and preferably 2% to 5% of the total composition.

**Therapeutic agents** may include, but are not limited to, flurbiprofen, fentanyl, ibuprofen, aspirin, paracetamol, benzocaine, lidocaine, menthol, hexylresorcinol, phenol, ambroxol hydrochloride, dextromethorphan hydrobromide, and guaifenesin, or any combinations thereof. The oral product may contain one or more therapeutic agents. In one embodiment, the one or more therapeutic agents may be present in a total amount of 0.1% to 10% and preferably 2% to 5% of the total composition.

The amount of each excipient, if present, and the total amount of all excipients will vary depending on the oral product and the active ingredient. The excipients should be present in the oral product in an amount which allows for processability, i.e. to form desired shapes. In one embodiment, the total amount of excipients may be 10% to 95%, preferably 40% to 90% and more preferably 50% to 80% of the total composition.

### Use

The user of the oral product may be a human or an animal. The user is preferably a human subject.

The oral product is administered to the oral cavity of the user. In the oral cavity, the oral product is exposed to saliva as well as inhaled and exhaled air. The oral product may be chewed, sucked, or slowly dissolved in the oral cavity of the user. The active ingredient is released in the oral cavity, and acts systemically or locally in the oral cavity including the whole respiratory tract, preferably in the upper airways and in the exhaled air. The active ingredient is released upon contact with saliva and/or upon contact with the inhaled and exhaled air.

The respiratory tract includes the upper respiratory tract and the lower respiratory tract. The upper respiratory tract includes the mouth, nasal cavity, and the throat (pharynx). The lower respiratory tract includes the trachea, primary bronchi, and the lungs.

The oral products allow the user to take a desired dose of antiviral active ingredients in a single dosage form.

The oral product can be conveniently used because it can be taken anywhere, for example on public transport or in shops. The oral product can be used whilst the user is wearing a face mask to further protect the user and third persons from infections such as COVID-19.

The oral product is kept in the oral cavity for a period of time sufficient to allow release of an effective amount of active ingredient. In one embodiment, when the oral product is a chewing gum, the chewing gum is chewed for at least 2, 5, 10, 15, 20, 40 or 60 minutes, most preferably 30 minutes. In one embodiment, the chewing gum releases at least 50%, and more preferably at least 70% of the active ingredient present in the chewing gum into the oral cavity.

A lozenge or pastille and the remaining oral products of the present invention may be capable of being dissolved in the oral cavity in about 5 to about 15 minutes, depending on the user's sucking pattern, thereby releasing the complete amount of the active ingredient to the oral cavity.

The oral product of the present invention can be taken several times a day as needed.

The oral product of the present invention is for use in a method of treatment or prevention of an infection with a coronavirus, an influenza virus, a coxsackievirus, or a herpes virus, wherein the method comprises the administration of the oral product to the oral cavity, the active ingredient is released from the oral product into the oral cavity, and the treatment or prevention involves reducing the virus load in the respiratory tract, preferably in the upper airways, and in the exhaled air.

In a preferred embodiment, the oral product of the present invention is for use in a method of treatment or prevention of an infection with a coronavirus, an influenza virus, a coxsackievirus, or a herpes virus, wherein the corona virus is selected from HCoV-NL63, HCoV-229E, HCoV-OC43, HKU1, SARS-CoV, SARS-CoV-2, and MERS-CoV; the influenza virus is influenza virus A or B; the coxsackievirus is group A coxsackievirus or group B coxsackievirus; and the herpes virus is herpes simplex virus (HSV), varicella-zoster virus (VZV) or human cytomegalovirus (HCMV).

In one embodiment, the product is kept in the oral cavity for chewing or sucking or slowly dissolving in the oral cavity.

### Advantageous effects

The oral products of the present invention offer an advantage over the prior art because it provides a convenient delivery vehicle that improves patient compliance and that can provide a desired amount of the antiviral active ingredient to the user.

The oral products of the present invention are for use in a method of treatment or prevention of an infection with a coronavirus, an influenza virus, a coxsackievirus, or a herpes virus.

The prevention of an infection with a coronavirus, an influenza virus, a coxsackievirus, or a herpes virus may be the prevention of the spreading of the infection from one subject to one or more further subject(s) or the prevention of an infection of a subject.

The active ingredient acts locally in the oral cavity and the entire respiratory tract including the exhaled air or systemically after absorption through the oral mucosa, for example via buccal or sublingual absorption routes. The active ingredient most preferably acts locally in the upper airways.

The oral products of the present invention release the active ingredient in liquid and/or in aerosol form on exposure to salvia in the oral cavity of the user. The active ingredient is further released by chewing, sucking or dissolving the oral product in the oral cavity.

Mechanical action, for example chewing, of the oral product can facilitate the release of the active ingredient and other additives such as sweeteners and flavourants. The oral product can become more pliable during use which improves the release of the active ingredient. An oral product having a filler that can dissolve or disintegrate during use may increase the pliability of the oral product.

Chewing action creates a pressure within the oral cavity of the user which may force the active ingredient directly into the systemic circulation of the user through the oral mucosa. The oral mucosa allows for rapid drug absorption as it has a thin epithelium and a rich vascularity.

Through ventilation of the oral cavity, the antiviral active ingredients reach the entire respiratory tract. The active ingredients cause a reduction of the virus load in the oral cavity and the entire respiratory tract, preferably in the upper airways. In a particular preferred embodiment, the virus load in the mouth and the exhaled air of a subject is reduced. In addition, viral proliferation may be reduced or even stopped. This contributes to the prevention of spreading an infection due to human-to-human transmission by aerosols from exhaled air. The reduction of the virus load in the oral cavity and respiratory tract in an infectious person reduces the risk of infecting others. Also, the antiviral activity of the active ingredients in the complete respiratory tract including the upper and the lower airways reduces the infection risk in a user when inhaling infectious air containing viruses from another subject.

The above described effects are particularly important in a method of treatment or prevention an infection with a coronavirus, wherein the coronavirus is SARS-CoV-2, as SARS-CoV-2 has high transmissibility and infectivity between humans.

The oral products of the present invention may provide a rapid release of the active ingredients. A rapid release of active ingredients may be desirable for a user when coming into contact with potentially infected subjects.

Alternatively, the oral products of the present invention may release the active ingredient over an extended period of time, such as for example over a period of one hour.

The oral products of the present invention may be administered before or after the exposure to a virus. When administered before the exposure to a virus, the active ingredient(s) due to their antiviral activity contribute to the above-described prevention of an infection. When administered after the exposure to a virus, the active ingredient(s) due to their antiviral activity can be used in the treatment of a viral infection either in the mouth or systemically.

The oral products of the present invention may comprise an antiviral active ingredient which is an essential oil. The essential oil treats, kills and/or inhibits proliferation of viral pathogens in the user. In addition, the essential oil may be used to provide flavour to the oral product. Moreover, essential oils usually are volatile which allows for effectively exerting their antiviral effect. That is, the effect of an essential oil is trifold in the present case. In a preferred embodiment, the oral products of the present invention also comprise an essential oil as antiviral active ingredient

In one embodiment, the essential oils are provided with a surfactant, such as sodium lauryl sulfate, which improves the solubilization of the essential oils in the saliva thereby enhancing their vaporisation.

In one embodiment, the oral product comprising an essential oil further comprises an adsorbent. In one embodiment, the oral product comprising an essential oil further comprises a natural extract.

It is further advantageous for the oral product of the present invention to include a flavourant. An oral product having a desirable taste may increase the contact time with the oral mucosa of the user.

In a preferred embodiment of the present invention, the oral product comprises the active ingredients and one or more sweeteners. In another embodiment, the oral product comprises the active ingredients, one or more sweeteners and an adsorbent.

In one preferred embodiment, the oral product comprises the active ingredients, one or more sweeteners, an adsorbent and a vitamin. In another preferred embodiment, the oral product comprises the active ingredients, one or more sweeteners, a vitamin and a mineral.

The oral product for use in a method of treatment or prevention of an infection with coronavirus, in particular COVID-19, preferably includes spermidine, quercetin, isoquercetin, propolis, manuka-honey, an essential oil, for example sage essential oil.

The oral product of the present invention for use in a method of treatment or prevention of an infection with coronavirus includes spermidine as spermidine provides a neuroprotective effect as for example described in WO93/12777 (cf. above), which can help to prevent neural damage which may be caused by COVID-19.

The oral product of the present invention for use in a method of treatment or prevention of an infection with coronavirus, is preferably a chewing gum or a lozenge, most preferably a chewing gum.

### Method of Manufacture

The oral products may be prepared using standard techniques and equipment known to those skilled in the art.

The method comprises mixing the antiviral active ingredient and one or more excipients. The active ingredient may be preblended to ensure uniform distribution throughout the oral prod uct.

In one embodiment, the process to prepare a chewing gum according to the invention involves melting or softening the gum base, incorporating the antiviral active ingredient and optionally one or more excipients selected from the group consisting elastomers, plasticisers, softeners, fillers, waxes, lubricants, buffering agents, stabilising agents, gelling agents, preservatives, adsorbants, and additives, while mixing and forming the batch into individual chewing gum pieces. The optional excipients are added to the batch in a manner well known to those of ordinary skill in the art. The ingredients may be added sequentially. The mixing and heating apparatus is well known in the chewing gum manufacturing arts. An example mixer is a brabender mixer. Individual chewing gum pieces may be formed using standard techniques known in the chewing gum art. Those skilled in the art will recognise that many variations of the described procedure may be followed. The chewing gum of the present invention has mechanical properties similar to conventional chewing gums.

In one embodiment, the method of preparing a chewing gum according to the invention comprises providing a portion of active ingredient, admixing the active ingredient with a chewing gum base and optional excipients, then compressing the admixture to obtain a compressed chewing gum, optionally with the addition of magnesium stearate as compressing aid.

In one embodiment, the chewing gum comprises 10-80%, preferably 20-60% gum base, 0.1-60%, preferably 10-40% sweetening agents, 0.1-10%, preferably 1-2% softeners or plasticisers, 1-2% flavourants, and 0.1-12%, preferably 1-10% active ingredients, based on the total composition of the chewing gum.

In another embodiment, the chewing gum comprises 40-80% gum base, 10-30% antiviral active ingredients and 10-30% other excipients, and preferably 60-80% gum base, 10-20% antiviral active ingredients and 10-20% other excipients, based on the total composition of the chewing gum.

In a preferred embodiment, the chewing gum comprises 60-80% gum base, 10-20% antiviral active ingredients, and optionally one or more of 0.1-10% sweetener, 0.0001-0.1% vitamin, 0.2% to 2% mineral, and 1-15% adsorbant, based on the total composition of the chewing gum.

In one embodiment, the ratio of gum base to the total of antiviral active ingredient(s) in the chewing gum is 6:1 to 10:1, and preferably 8:1.

When used as compressing aid magnesium stearate is added in an amount of 0.5% to 10%, preferably 1% to 5% and most preferably 2%to3% based on the total composition of the chewing gum.

In one embodiment, the process to prepare the lozenge according to the invention comprises thoroughly mixing active ingredient and the optional excipients and then compressing the mixture, optionally with the addition of magnesium stearate as compressing aid.

Similarly, the pastille, gel, tablet, hard boilies, and other candy-like compositions of the present invention may be prepared following established standard procedures known to those skilled in the art.

### Example

### Preparation Example:

A chewing gum was prepared from the following components:

| **Ingredient** | **Weight %** |
|---|---|
| Wavelet Base | 39.9124 |
| Health in Gum 03 | 39.9124 |
| Lemon oil | 2.5000 |
| Magnesium stearate | 2.0000 |
| Silicon dioxide | 6.0000 |
| Sucralose | 0.1250 |
| Quercetin | 1.5000 |
| Zinc bisglycinate | 0.5000 |
| Vitamin D | 0.0002 |
| Panax Ginseng Root Powder | 2.0000 |
| Spermidine | 0.0500 |
| Cinnamon Oil | 1.5000 |
| Ginger Oil | 2.5000 |
| Peppermint Oil | 1.5000 |

### Effect of the gum:

The effect of the gum on the viral load in exhaled air was tested on 9 patients infected with SARS-COV-2 (alpha variant: 3 patients; delta variant: 2 patients).

The gum was chewed by the patient for 15 minutes and then discarded. For obtaining samples, patients exhaled a pre-defined volume of air through an uncontaminated air particle filter into a lung function device. Samples were taken before and 0, 15, 30, 60, 90, 120 and 180 minutes after chewing the gum. For each point in time, the viral load of SARS-COV-2 on the air particle filter was determined by means of a PCR test.

The following table shows the reduction [%] in comparison to the viral load before chewing the gum:

| Patient | 0min | 15min | 30min | 45min | 60min | 90min | 180min |
|---|---|---|---|---|---|---|---|
| 1 | | 96 | 89 | 87 | 97 | 96 | |
| 2 | 24 | 88 | 92 | 75 | 93 | 88 | 73 |
| 3 | 50 | 82 | 90 | 91 | 98 | 96 | 91 |
| 4 | 92 | 86 | 100 | 88 | 100 | 0 | |
| 5 | 86 | 100 | 100 | 100 | 100 | 100 | |
| 6 | 57 | 80 | 85 | 58 | 47 | 0 | |
| 7 | 94 | 92 | 79 | 68 | | 0 | 0 |
| 8 | 85 | 78 | 81 | 100 | 91 | 62 | |
| 9 | 98 | 88 | 95 | 96 | 85 | 65 | |
| Average Reduction | 73 | 88 | 90 | 85 | 89 | 56 | |

The chewing gum according to the invention significantly decreased the amount of virus which was exhaled by the patients. On average, viral load was reduced by approx. 90% at 15, 30, 45 and 60 minutes after chewing. At 60 minutes, no virus could be detected in the exhaled air of two patients. Even after 90 minutes, viral load was reduced by 56%. The effect persisted for up to 3 hours.

Based on these results, it is expected that the oral product according to the invention prevents or reduces spread of SARS-COV-2 and other viruses by speaking, coughing, laughing and singing.

## Claims

1. An oral product comprising an antiviral active ingredient which includes a combination of spermidine and one or more polyphenols,
for use in a method of treatment or prevention of an infection with a coronavirus, an influenza virus, a coxsackievirus, or a herpes virus, wherein
the method comprises the administration of the oral product to the oral cavity,
the active ingredient is released from the oral product into the oral cavity, and the treatment or prevention involves reducing the virus load in the respiratory tract, and in the exhaled air.

2. The oral product for use according to claim 1, wherein the product is kept in the oral cavity for chewing or sucking or slowly dissolving in the oral cavity.

3. The oral product for use according to claim 1 or 2, wherein the treatment or prevention involves reducing the virus load in the in the upper airways and in the exhaled air.

4. The oral product for use according to any one of claims 1 to 3, wherein the oral product is a product selected from mouth soluble or dispersible forms; suckable, eatable, chewable or coherent forms; or forms rapidly disintegrating in the mouth.

5. The oral product for use according to any one of claims 1 to 4, wherein the polyphenol is quercetin and/or isoquercetin.

6. The oral product for use according to any one of claims 1 to 5, wherein the antiviral active ingredient is a combination of
• spermidine,
• polyphenols,
• propolis, and
• manuka-honey.

7. The oral product for use according to any one of claims 1 to 6 , wherein the antiviral active ingredient further includes natural extracts and/or essential oils.

8. The oral product for use according to claim 7, wherein the natural extract and/or the essential oil are one or more of a natural extract and/or an essential oil from tea tree, sage, cinnamon, eucalyptus, oregano, thyme, basil, cassia, lime, lemon, peppermint, ginger, ginseng, melissa, clove or lavender, preferably from tea tree, sage, cinnamon, ginger, ginseng, eucalyptus, peppermint, and clove, and more preferably from lemon, cinnamon, ginger and peppermint, or any combinations thereof.

9. The oral product for use according to any one of claims 1 to 8, further comprising one or more excipients selected from elastomers, plasticisers, softeners, fillers, waxes, lubricants, buffering agents, stabilising agents, gelling agents, preservatives, adsorbants, and additives; optionally wherein the additives are selected from sweetening agents, flavourants, colourants, taste masking agents, oral care agents, minerals, vitamins, amino acids, antioxidants, therapeutic agents, or any combinations thereof.

10. The oral product for use according to any one of claims 1 to 10, wherein the oral product is selected from a chewing gum, a lozenge, a pastille, a gel, a tablet, and hard boilies.

11. The oral product for use according to claim 10, wherein the oral product is a chewing gum.

12. The oral product for use according to claim 11, wherein the chewing gum further comprises a gum base, and optionally one or more excipients selected from plasticisers, elastomers, softeners, fillers, and bulking agents, or wherein the oral product is a lozenge, wherein the lozenge further comprises one or more excipients selected from sweetening agents and flavourants.

13. The oral product for use according to claim 12, wherein the oral product is a chewing gum comprising 60-80% gum base, 10-20% antiviral active ingredients, and optionally one or more of 0.1-10% sweetener, 0.0001-0.1% vitamin, 0.2% to 2% mineral, and 1-15% adsorbant.

## Patentansprüche

1. Orales Produkt mit einem antiviralen Wirkstoff, der eine Kombination von Spermidin und einem oder mehreren Polyphenolen enthält,
zur Verwendung in einem Verfahren zur Behandlung oder Vorbeugung einer Infektion mit einem Coronavirus, einem Influenzavirus, einem Coxsackie-Virus oder einem Herpesvirus, wobei
das Verfahren die Verabreichung des oralen Produkts in die Mundhöhle umfasst,
der Wirkstoff aus dem oralen Produkt in die Mundhöhle freigesetzt wird, und
die Behandlung oder Vorbeugung das Verringern der Viruslast in den Atemwegen und in der ausgeatmeten Luft umfasst.

2. Orales Produkt zur Verwendung gemäß Anspruch 1, wobei das Produkt in der Mundhöhle zum Kauen oder Lutschen oder zum langsamen Auflösen in der Mundhöhle gehalten wird.

3. Orales Produkt zur Verwendung gemäß Anspruch 1 oder 2, wobei die Behandlung oder Vorbeugung das Verringern der Viruslast in den oberen Atemwegen und in der ausgeatmeten Luft umfasst.

4. Orales Produkt zur Verwendung gemäß einem der Ansprüche 1 bis 3, wobei das orale Produkt ein Produkt ist, das aus mundlöslichen oder dispergierbaren Formen; lutschbaren, essbaren, kaubaren oder kohärenten Formen; oder schnell im Mund zerfallenden Formen ausgewählt ist.

5. Orales Produkt zur Verwendung gemäß einem der Ansprüche 1 bis 4, wobei das Polyphenol Quercetin und/oder Isoquercetin ist.

6. Orales Produkt zur Verwendung gemäß einem der Ansprüche 1 bis 5, wobei der antivirale Wirkstoff eine Kombination von
• Spermidin,
• Polyphenolen,
• Propolis und
• Manuka-Honig
ist.

7. Orales Produkt zur Verwendung gemäß einem der Ansprüche 1 bis 6, wobei der antivirale Wirkstoff weiterhin natürliche Extrakte und/oder ätherische Öle umfasst.

8. Orales Produkt zur Verwendung gemäß Anspruch 7, wobei der natürliche Extrakt und/oder das ätherische Öl eines oder mehrere von einem natürlichen Extrakt und/oder einem ätherischen Öl aus Teebaum, Salbei, Zimt, Eukalyptus, Oregano, Thymian, Basilikum, Kassie, Limette, Zitrone, Pfefferminze, Ingwer, Ginseng, Melisse, Nelke oder Lavendel, bevorzugt aus Teebaum, Salbei, Zimt, Ingwer, Ginseng, Eukalyptus, Pfefferminze und Nelke, und mehr bevorzugt aus Zitrone, Zimt, Ingwer und Pfefferminze, oder beliebige Kombinationen davon sind.

9. Orales Produkt zur Verwendung gemäß einem der Ansprüche 1 bis 8, ferner umfassend einen oder mehrere Hilfsstoffe, ausgewählt aus Elastomeren, Plastifizierungsmitteln, Weichmachern, Füllstoffen, Wachsen, Gleitmitteln, Puffersubstanzen, Stabilisierungsmitteln, Geliermitteln, Konservierungsmitteln, Adsorptionsmitteln und Zusatzstoffen; wobei die Zusatzstoffe optional aus Süßungsmitteln, Geschmacksstoffen, Farbstoffen, geschmacksmaskierenden Mitteln, Mundpflegemitteln, Mineralien, Vitaminen, Aminosäuren, Antioxidantien, therapeutischen Mitteln oder beliebigen Kombinationen davon ausgewählt sind.

10. Orales Produkt zur Verwendung gemäß einem der Ansprüche 1 bis 10, wobei das orale Produkt aus einem Kaugummi, einer Lutschtablette, einer Pastille, einem Gel, einer Tablette und harten Boilies ausgewählt ist.

11. Orales Produkt zur Verwendung gemäß Anspruch 10, wobei das orale Produkt ein Kaugummi ist.

12. Orales Produkt zur Verwendung gemäß Anspruch 11, wobei der Kaugummi weiterhin eine Gummibasis und optional einen oder mehrere Hilfsstoffe, ausgewählt aus Plastifizierungsmitteln, Elastomeren, Weichmachern, Füllstoffen und Füllmitteln, umfasst, oder wobei das orale Produkt eine Lutschtablette ist, wobei die Lutschtablette weiterhin einen oder mehrere Hilfsstoffe, ausgewählt aus Süßungsmitteln und Geschmacksstoffen, umfasst.

13. Orales Produkt zur Verwendung gemäß Anspruch 12, wobei das orale Produkt ein Kaugummi ist, der 60-80% Gummibasis, 10-20% antivirale Wirkstoffe und optional eines oder mehrere von: 0,1-10% Süßungsmittel, 0,0001-0,1% Vitamin, 0,2% bis 2% Mineralien und 1-15% Adsorptionsmittel umfasst.

## Revendications

1. Produit oral comprenant un principe actif antiviral qui inclut une combinaison de spermidine et d'un ou plusieurs polyphénols,
pour une utilisation dans un procédé de traitement ou de prévention d'une infection par un coronavirus, un virus de la grippe, un virus Coxsackie, ou un virus de l'herpès, dans lequel
le procédé comprend l'administration du produit oral dans la cavité orale,
le principe actif est libéré du produit oral dans la cavité orale, et
le traitement ou la prévention implique la réduction de la charge virale dans les voies respiratoires, et dans l'air expiré.

2. Produit oral pour utilisation selon la revendication 1, dans lequel le produit est conservé dans la cavité orale pour être mâché ou sucé ou dissous lentement dans la cavité orale.

3. Produit oral pour utilisation selon la revendication 1 ou la revendication 2, dans lequel le traitement ou la prévention implique la réduction de la charge virale dans les voies aériennes supérieures et dans l'air expiré.

4. Produit oral pour utilisation selon l'une quelconque des revendications 1 à 3, dans lequel le produit oral est un produit choisi parmi des formes solubles ou dispersibles dans la bouche ; formes à sucer, à manger, à mâcher ou homogènes ; ou formes se désintégrant rapidement dans la bouche.

5. Produit oral pour utilisation selon l'une quelconque des revendications 1 à 4, dans lequel le polyphénol est de la quercétine et/ou de l'isoquercétine.

6. Produit oral pour utilisation selon l'une quelconque des revendications 1 à 5, dans lequel le principe actif antiviral est une combinaison de
• spermidine,
• polyphénols,
• propolis, et
• miel de manuka.

7. Produit oral pour utilisation selon l'une quelconque des revendications 1 à 6, dans lequel le principe actif antiviral comporte en outre des extraits naturels et/ou des huiles essentielles.

8. Produit oral pour utilisation selon la revendication 7, dans lequel l'extrait naturel et/ou l'huile essentielle sont un ou plusieurs parmi un extrait naturel et/ou une huile essentielle provenant d'arbre à thé, de sauge, de cannelle, d'eucalyptus, d'origan, de thym, de basilic, de casse, de citron vert, de citron, de menthe poivrée, de gingembre, de ginseng, de mélisse, de clou de girofle ou de lavande, de préférence provenant d'arbre à thé, de sauge, de cannelle, de gingembre, de ginseng, d'eucalyptus, de menthe poivrée et de clou de girofle, et plus préférablement provenant de citron, de cannelle, de gingembre et de menthe poivrée, ou toute combinaison de ceux-ci.

9. Produit oral destiné pour utilisation selon l'une quelconque des revendications 1 à 8, comprenant en outre un ou plusieurs excipients choisis parmi des élastomères, des plastifiants, des adoucissants, des charges, des cires, des lubrifiants, des agents tampons, des agents stabilisants, des agents gélifiants, des conservateurs, des adsorbants et des additifs ; facultativement dans lequel les additifs sont choisis parmi des agents édulcorants, des arômes, des colorants, des agents de masquage du goût, des agents de soins bucco-dentaires, des minéraux, des vitamines, des acides aminés, des antioxydants, des agents thérapeutiques, ou toute combinaison de ceux-ci

10. Produit oral pour utilisation selon l'une quelconque des revendications 1 à 10, dans lequel le produit oral est choisi parmi un chewing-gum, une tablette, une pastille, un gel, un comprimé, et des bouillettes dures.

11. Produit oral pour utilisation selon la revendication 10, dans lequel le produit oral est un chewing-gum.

12. Produit oral pour utilisation selon la revendication 11, dans lequel le chewing-gum comprend en outre une base de gomme, et facultativement un ou plusieurs excipients choisis parmi des plastifiants, des élastomères, des adoucissants, des charges, et des agents de gonflement, ou dans lequel le produit oral est une tablette, dans lequel la tablette comprend en outre un ou plusieurs excipients choisis parmi des agents édulcorants et des arômes.

13. Produit oral pour utilisation selon la revendication 12, dans lequel le produit oral est un chewing-gum comprenant 60 à 80 % de base de gomme, 10 à 20 % d'ingrédients actifs antiviraux, et facultativement un ou plusieurs parmi 0,1 à 10 % d'édulcorant, 0,0001 à 0,1 % de vitamine, 0,2 % à 2 % de minéral, et 1 à 15 % d'adsorbant.
